# EUROPEAN PATENT SPECIFICATION

(11) **EP 2 687 191 B1**
(45) Date of publication and mention of the grant of the patent: **02.03.2016**
(21) Application number: 12757696.5
(22) Date of filing: 06.03.2012
(51) Int. Cl.: A61F 2/56, A61F 2/68, A61F 5/01, A63B 23/16, B25J 9/14, F15B 15/10, G06F 3/01

(54) **GLOVE-TYPE POWER ASSIST DEVICE**
BEWEGUNGSHILFSVORRICHTUNG IN HANDSCHUHFORM
DISPOSITIF ASSISTÉ DE TYPE GANT

(30) Priority: 16.03.2011 JP 2011057791
(43) Date of publication of application: 22.01.2014
(73) Proprietor: Daiya Industry Co., Ltd., Okayama-shi, Okayama 701-0204 (JP); National University Corporation Okayama University, Kita-ku Okayama-shi Okayama 700-8530 (JP)
(72) Inventor: NORITSUGU, Toshiro, Okayama-shi, Okayama 700-8530 (JP); SASAKI, Daisuke, Okayama-shi, Okayama 700-8530 (JP); OGAWA, Kazunori, Okayama-shi, Okayama 701-0204 (JP); IKEDA, Tomohiro, Okayama-shi, Okayama 701-0204 (JP)
(74) Representative: Müller-Boré & Partner Patentanwälte PartG mbB
(86) International application number: PCT/JP2012/055694
(87) International publication number: WO 2012/124546

(56) References cited:
- JP-A- 2001 276 101
- JP-A- 2006 000 294
- JP-A- 2006 000 294
- US-A- 4 739 692
- US-B1- 6 168 634
- KYUNG-WON MOON ET AL: "Development of a Slim Haptic Glove Using McKibben Artificial Muscles", SICE-ICCAS 2006 INTERNATIONAL JOINT CONFERENCE, IEEE, PISCATAWAY, NJ, USA, 1 October 2006 (2006-10-01), pages 204-208, XP031050583, ISBN: 978-89-950038-4-8
- D. SASAKI ET AL: "Wearable power assist device for hand grasping using pneumatic artificial rubber muscle", RO-MAN 2004. 13TH IEEE INTERNATIONAL WORKSHOP ON ROBOT AND HUMAN INTERACTIVE COMMUNICATION (IEEE CATALOG NO.04TH8759), 1 January 2004 (2004-01-01), pages 655-660, XP055137590, DOI: 10.1109/ROMAN.2004.1374840 ISBN: 978-0-78-038570-2
- KIMINORI TOYA ET AL: "Power-Assist Glove Operated by Predicting the Grasping Mode", JOURNAL OF SYSTEM DESIGN AND DYNAMICS, vol. 5, no. 1, 1 January 2011 (2011-01-01) , pages 94-108, XP055137491, DOI: 10.1299/jsdd.5.94
- TOSHIRO NORITSUGU ET AL: "Power Assist Wear Driven with Pneumatic Rubber Artificial Muscles", 2008 15TH INTERNATIONAL CONFERENCE ON MECHATRONICS AND MACHINE VISION IN PRACTICE, 1 December 2008 (2008-12-01), pages 539-544, XP055137594, DOI: 10.1109/MMVIP.2008.4749589 ISBN: 978-1-42-443779-5

## Description

### TECHNICAL FIELD

The present invention relates to glove-type power assist device putted on a hand and assisting finger motions of wearer's hand.

### BACKGROUND ART

A shortage of care personnel becomes more serious as a birth rate falls and an aging population increases. The elderly caring the elderly, so called elder-to-elder caring, is often heard. Power assist devices become highly demanded, which relieve a physical strain on carers or support those who need care to be independent. Various power assist devices are proposed depending on parts to be assisted. Power assist devices for assisting folding motions of fingers of a hand is already known too.

For example, Patent Document 1 discloses a power assist device (glove-type power assist device) including; tubular actuators being bent by raising an inside pressure, the inside pressure being raised by a provision of a fluid; a wearing body (glove) for fixing the actuators along finger joints of a hand.
Patent Document 1 also discloses providing fluid receiving portions for the actuators along a longitudinal direction of the actuators, and providing preventing means preventing a stretch along the longitudinal direction on a side of the inside of the actuators.

The glove-type power assist device of Patent Document 1 is not only suitable for assisting motions which accompany large displacements but also possible to alleviate a bound feeling given to wearers by having bending centers of the actuators and folding centers of finger joints of a hand corresponded. Although the bending centers of the actuator and the folding centers of finger joints of a hand correspond in the glove-type power assist device of Patent Document 1, since it undergoes the procedure that the bending centers of the actuators gradually approach to the folding centers of finger joints when the actuators deform, an uncomfortable feeling of which the wearers feel around the finger joints of the hand is not completely removed. The power assist glove-type device of Patent Document 1 is not necessarily easy to make it compact and portable.

Patent Document 1: Japanese Patent Application Publication No. 2006-000294 (Claims 1, 12, 13, paragraphs 0023, 0051, Figures. 17 to 19)
KYUNG-WON MOON ET AL: "Development of a Slim Haptic Glove Using McKibben Artificial Muscles", SICE-ICCAS 2006 INTERNATIONAL JOINT CONFERENCE, IEEE, PISCATAWAY, NJ, USA, 1 October 2006 (2006-10-01), pages 204-208, discloses a pneunmatic haptic glove system which is designed for virtual environments. Artificial muscles are used. The Artificial muscles are actuated by hydraulic system.
D. Sasaki ET AL: "Wearable power assist device for hand grasping using pneumatic artificial rubber muscle", RO-MAN 2004. 13th IEEE International Workshop on Robot and Human Interactive Communication (IEEE Catalog No.04TH8759), 1 January 2004 (2004-01-01), pages 655-660, discloses a power assist device for hand grasping using pneumatic artificial rubber muscle.

Kiminori Toya ET AL: "Power-Assist Glove Operated by Predicting the Grasping Mode", Journal of System Design and Dynamics, vol. 5, no. 1, 1 January 2011 (2011-01-01), pages 94-108, discloses a control algorithm for a power-assist glove. The control algorithm estimates human grasping intention.
Toshiro Noritsugu ET AL: "Power Assist Wear Driven with Pneumatic Rubber Artificial Muscles", 2008 15th International Conference on Mechatronics and Machine Vision in Practice, 1 December 2008 (2008-12-01), pages 539-544, discloses a power assist wear driven with pneumatic rubber artificial muscles.

### DISCLOSURE OF INVENTION

### Problem to be Solved by the Invention

The present invention provides a glove-type power assist device that is easy to use in daily lives, easy to wear, and does not give an unconfutable feeling when used. Specifically, the present invention provides a glove-type power assist device that does not undergo the procedure that the bending centers of the actuators gradually approach to the folding centers of finger joints as the actuators deform, but does undergo the procedure that the bending centers of the actuators correspond to the folding centers of finger joints from the beginning when actuators begin to bend to smoothly assist finger motions of a hand of a wearer without giving a large strain on the finger joints of the wearer and further to completely remove an uncomfortable feeling that the wearer feels around the finger joints of the hand. The present invention also provides the glove-type power assist device that is easy to make it compact and portable.

### Means to Solve the Problems

Above said problems are solved by providing a glove-type power assist according to claim 1. It includes a glove provided with finger inserting portions for inserting fingers of a hand; expanding and contracting tubes fixed to back side of the finger inserting portions of the glove, a stretch along a longitudinal direction is restricted on a side fixed to the finger inserting portions, and a fluid supplying member supplying a fluid to the expanding and contracting tubes. The expanding and contracting tubes are bent to a palm-side by expanding the back side of the expanding and contracting tubes more than the palm-side to assist a folding motion of fingers inserted into the finger inserting portions. Substantially whole of the glove is configured by an elastic base material. Side lines (virtual lines along centers of sides from which the sides of each finger inserting portion are divided into a back side and a palm side) are provided as boundaries between the back side and the palm-side of the finger inserting portions. Each side line has an unstretchable configuration (including not only a configuration in which it does not stretch at all but also a configuration in which it slightly stretches) in which a stretch to the longitudinal direction of the finger inserting portions is prevented so that the expanding and contracting tubes can bend at folding points of wearer's finger joints as centers.

This enables for the expanding and contracting tubes to smoothly bend as the folding points of fingers joints as centers. Therefore, the wearer's finger motions are assisted without giving a large strain on the finger joints of the wearer of the glove-type power assist device. "Assist" is not only a concept which literally assisting a portion to be assisted (fingers of a hand) by applying a power along a forward direction with respect to a motion of the portion to be assisted but also includes a concept which assisting the portion to be assisted by applying a power along a reverse direction with respect to the motion of the portion to be assisted. The latter case is included in the concept of "assist" in order to make it clear that a glove-type load applying device used for rehabilitations or muscle trainings and a glove-type virtual experience device used for embodying virtual realities are included in a scope of the glove-type power assist device of the present invention.

In the glove-type power assist device of the present invention, the side lines of the finger inserting portions are provided on the boundary between the back side and the palm-side, as descripted above. More precisely, the side lines are provided in the position where the side lines overlap with lines connecting the folding points of wearer's finger joints when the finger inserting portions are laterally seen. According to this, the bending centers of the expanding and contracting tubes more precisely correspond to the folding centers of the finger joints of the hand of the wearer. Concrete names of finger joints to which the side lines of the finger inserting portions are overlapped are DIP joint (distal interphalangeal joint), PIP joint (proximal interphalangeal joint), and MP joint (metacarpophalangeal joint) of a fore finger, a middle finger, a ring finger and a little finger; IP joint (interpharangeal joint) and MP joint (metacarpal phalangeal joint) of a thumb.

In the glove-type power assisted device of the present invention, a concrete method for configuring the side lines unstretchable is not particularly limited. For example, the side lines of the finger inserting portions are made unstretchable configuration by fixing unstretchable liner members (including not only a configuration in which it does not stretch at all but also a configuration in which it slightly stretch) along the side lines of the finger inserting portions. As the unstretchable liner members, synthetic fibers such as polyester fibers or natural fibers such as cotton fibers are exemplified. The unstretchable liner members may be made of metal. The unstretchable liner members include not only strings but also band shaped objects. As methods for fixing the unstretchable liner members to the finger inserting portions, bonding or sewing is exemplified. In case the strings are used as the unstretchable liner members, the strings are to be sewed onto the finger inserting portions.

Further, it is preferable that unbending portions not overlapping the finger joints of the finger inserting portions and provided on the palm-side than the side lines are configured as non-slip portions covered with non-slip sheets. At the same time, bending portions overlapping the finger joints of the finger inserting portions and provided on the palm-side than the side lines are configured as blank portions not covered with the non-slip sheets and exposing the elastic base material. According to this, an object is securely grabbed in wearing state without spoiling an operability of the glove-type power assist device. It is preferable that the blank portions are provided so that they are triangular shapes (triangles having bottoms on palm-side of the finger inserting portions and summits near the side lines) when they are laterally seen. The number of the blank portions corresponds to the number of joints of each finger. Namely, two blank portions (for IP joint (a first joint) and MP joint (a second joint)) are provided on the finger inserting portion (thumb inserting portion described later). Three blank portions (for DIP joint (a first joint), PIP joint (second joint) and MP joint (third joint)) are provided on other finger inserting portions (the fore finger inserting portion and the three fingers commonly inserting portion described later).

Still further, according to the glove-type power assist device of the present invention, it is preferable that a cutline is provided on the glove to cover a portion from a base of a forefinger on a thumb side to an opening for wearing the glove, and a fastener is provided for fastening the cutline. This makes it easy to wear the glove-type power assist device. Especially, this makes it easy for those who have a handicap on fingers to wear the glove-type power assist device. In addition, this makes it easy to manufacture (sewing and others) the glove-type power assist device and maintain it. Various fasteners such as a slide fastener, a hook and loop fastener, and buttons and others are exemplified as the fastener for fastening the cutline. Among them, the slide fastener is preferably used as the fastener for fastening the cutline because it continuously fastens the cutline and it does not open unless it is intentionally slid.

A shape of the glove (how the finger inserting portions divide) is not particularly limited in the glove-type power assist device of the present invention. Although the finger inserting portions of the glove consist essentially of a thumb inserting portion for inserting a thumb of a wearer, a forefinger inserting portion for inserting a forefinger, a middle finger inserting portion for inserting a middle finger, a ring finger inserting portion for inserting the ring finger, and a little finger inserting portion for inserting a little finger, it is preferable that the middle finger inserting portion, the ring finger inserting portion, and the little finger inserting portion are put together to one fingers inserting portion (three fingers commonly inserting portion). An operability of the glove-type power assist device is enhanced and the glove-type power assist device is easily worn, if the finger inserting portions of the glove are consist essentially of the thumb inserting portion, the forefinger inserting portion, and the three fingers commonly inserting portion. In addition, costs required for the glove-type power assist device are reduced because the number of the expanding and contracting tubes fixed to the three fingers commonly inserting portion is reduced. In case the forefinger inserting portion, the middle finger inserting portion, the ring finger inserting portion and the little finger inserting portion are put together to one fingers inserting portion (four fingers commonly inserting portion) and the finger inserting portions of the glove consist essentially of the thumb inserting portion and the four fingers commonly inserting portion, it is more advantageous in an ease to wear the glove-type power assist device and in costs required for it. However, it has a defect in that an operability of the glove-type power assist device is spoiled.

Although a kind of the fluid supplying member is not particularly limited in the glove-type power assist device of the present invention, it is preferable that the fluid supplying member includes a tank whose capacity being equal to or below than 500ml and filled with a liquefied carbon dioxide, an on-off valve for opening and closing the tank, and a valve controlling portion for controlling the opening or closing status of the on-off valve. This makes the fluid supplying member compact and portable. Therefore, the glove-type power assist device is also become compact and portable. Volume of the tank is preferably equal to or below than 300ml, or is more preferably equal to or below than 200ml. On the other hand, if the volume is too small, liquefied carbon dioxide filled in the tank runs shortage in short time to often fill the liquefied carbon dioxide or to replace the tank. Therefore, the volume of tank is equal to or more than 10ml. The volume of the tank is preferably equal to or more than 30ml, or is more preferably equal to or more than 50ml.

### Effects of the Invention

As descripted above, according to the present invention, the glove-type power assist device is provided in which an uncomfortable feeling is removed when using, which is easily worn and easily used in daily lives. Specifically, the present invention provides a glove-type power assist device that does not undergo the procedure that the bending centers of the actuators gradually approach to the folding centers of finger joints as the actuators deform, but does undergo the procedure that the folding centers of the actuators correspond to the bending centers of finger joints from the beginning when actuators begin to bend to smoothly assist finger motions of a hand of wearers without giving a large strain on the finger joints of the wearer and further to completely remove a uncomfortable feeling that the wearer feels around the finger joints of the hand. The present invention also provides the glove-type power assist device that is easy to make it compact and portable.

### BRIEF DESCRIPTION OF THE DRAWINGS

Fig. 1 illustrates a glove-type power assist device seen from a back side, in which a fastener is fastened;
Fig. 2 illustrates the glove-type power assist device seen from the back side, in which the fastener is opened;
Fig. 3 illustrates the glove-type power assist device seen from a palm side;
Fig. 4 illustrates the glove-type power assist device seen from a thumb side, in which a forefinger inserting portion is straightened;
Fig. 5 illustrates the glove-type power assist device seen from a thumb side, in which a forefinger inserting portion is folded;
Fig. 6 illustrates an expanding and contracting tube laterally seen, which is in a contracting state (unbending state);
Fig. 7 is a cross sectional view of the expanding and contracting tube in the contracting state (unbending state) cut across a plane perpendicular to a center axis;
Fig. 8 illustrates the expanding and contracting tube laterally seen, which is in an expanding state (bending state); and
Fig. 9 is a cross sectional view of the expanding and contracting tube in the expanding state (bending state) cut across a plane perpendicular to a center axis.

### Embodiment FOR CARRYING OUT THE INVENTION

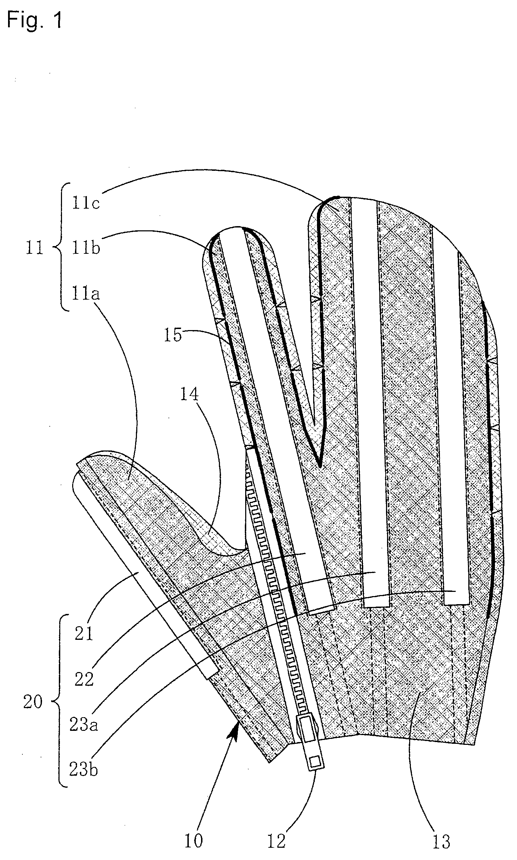

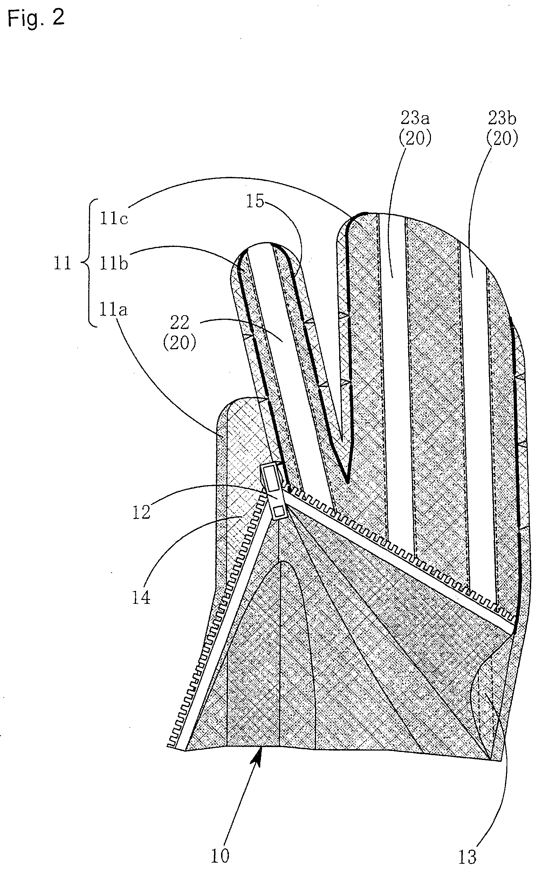

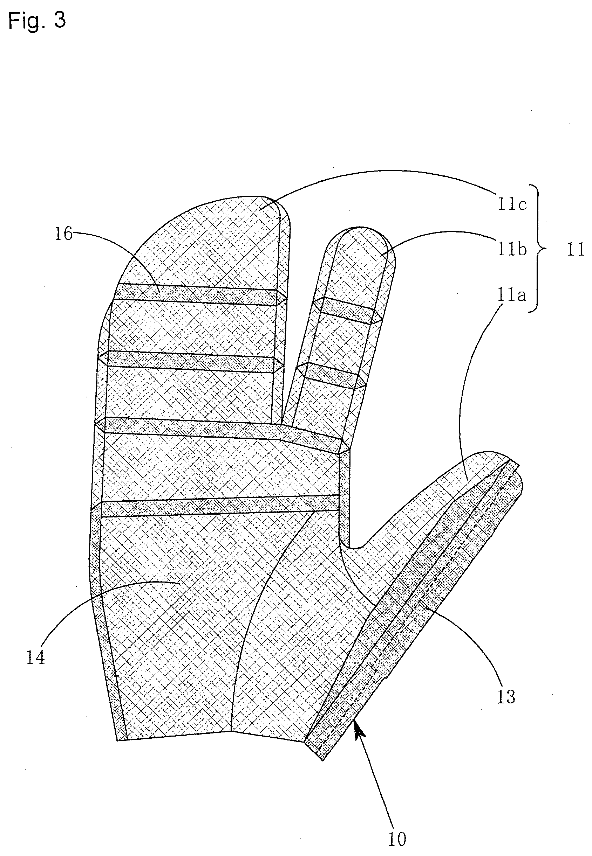

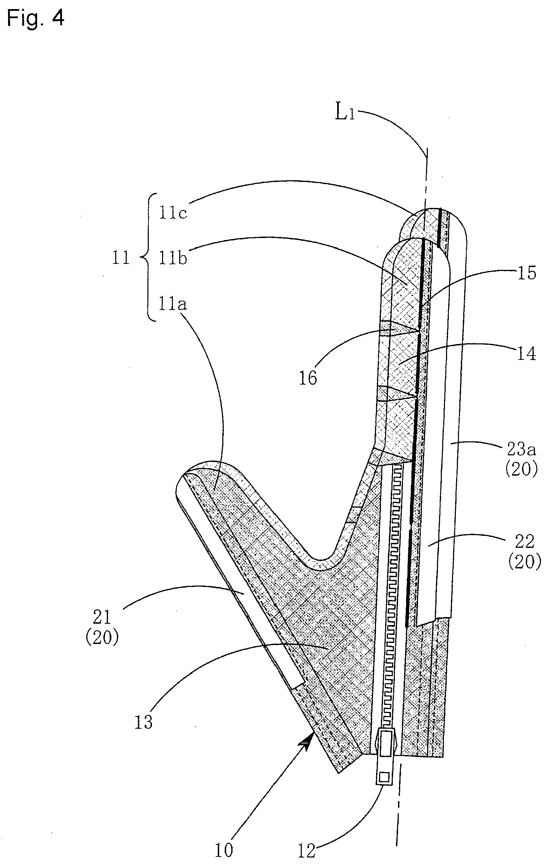

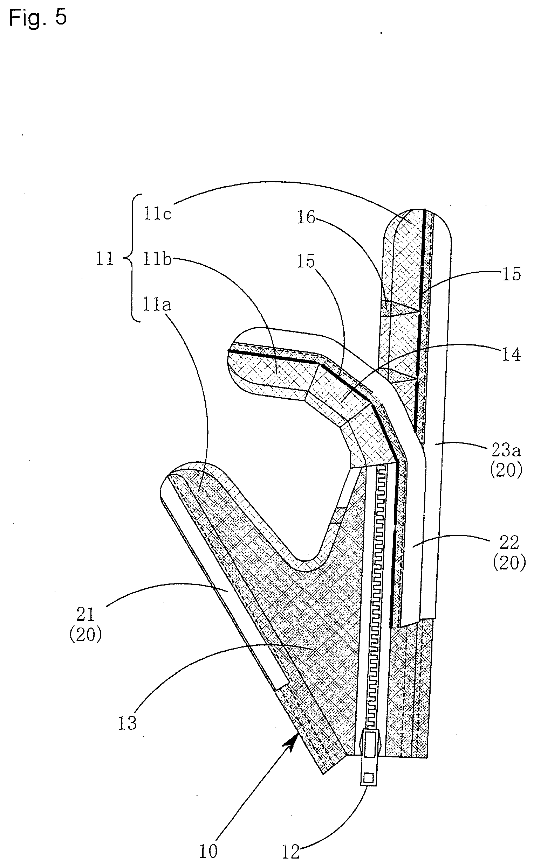

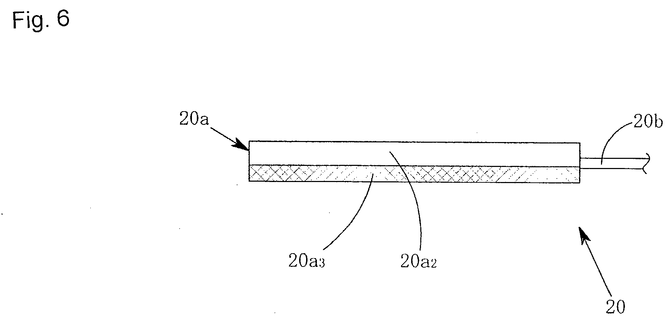

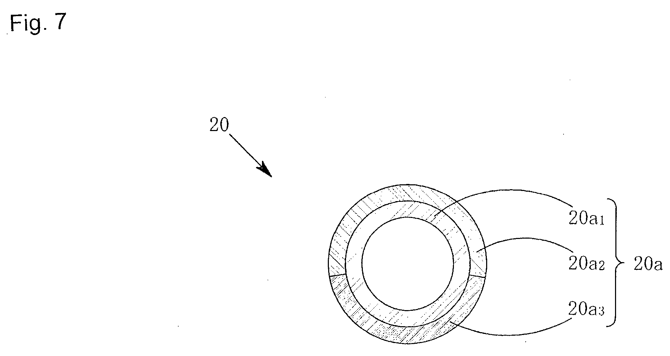

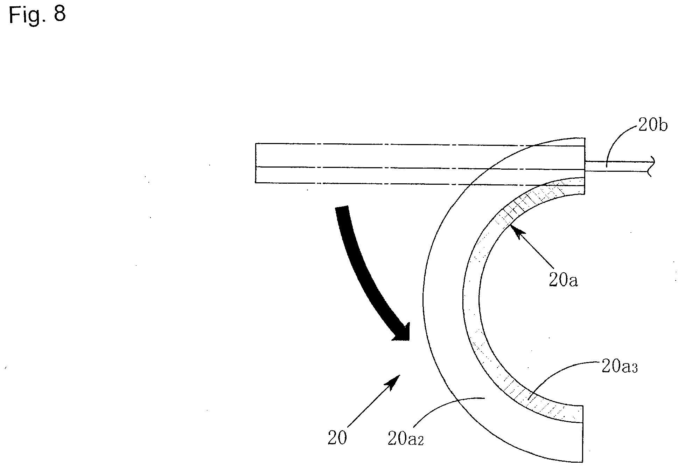

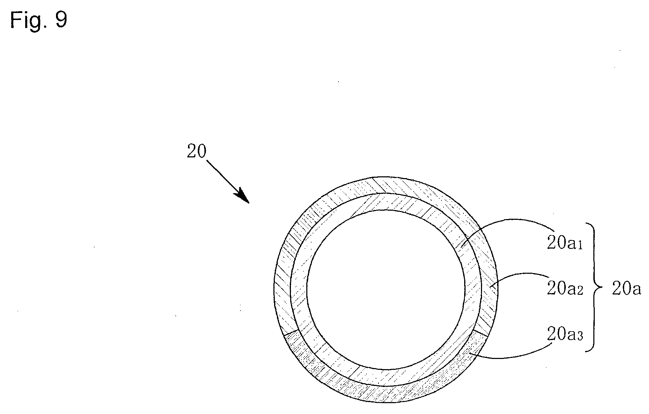

Preferable embodiments of the glove-type power assist device of the present invention with reference to Figures. Fig. 1 illustrates a glove-type power assist device seen from a back side, in which a fastener 12 is fastened. Fig. 2 illustrates the glove-type power assist device seen from the back side, in which the fastener 12 is opened. Fig. 3 illustrates the glove-type power assist device seen from a palm side. Fig. 4 illustrates the glove-type power assist device seen from a thumb side, in which a forefinger inserting portion 11b is straightened. Fig. 5 illustrates the glove-type power assist device seen from a thumb side, in which a forefinger inserting portion 11b is bent. Fig. 6 illustrates an expanding and contracting tube 20 laterally seen, which is in a contracting state (unbending state). Fig. 7 is a cross sectional view of the expanding and contracting tube 20 in the contracting state (unbending state) cut across a plane perpendicular to a center axis. Fig. 8 illustrates the expanding and contracting tube 20 laterally seen, which is in an expanding state (bending state). Fig. 9 is a cross sectional view of the expanding and contracting tube 20 in the expanding state (bending state) cut across a plane perpendicular to a center axis.

### [Outline]

The glove-type power assist device of the present embodiment includes a glove 10, the expanding and contracting tubes 20, and a fluid supplying member (not illustrated in Figures) as illustrated in Fig. 1. The glove 10 is provided with finger inserting portions 11 for inserting fingers of a hand. The expanding and contracting tubes 20 are fixed to back side (dorsum manus side) of the finger inserting portions 11 of the glove 10. If a fluid is provided to the expanding and contracting tubes 20 from the fluid supplying unit to raise an inside pressure, the expanding and contracting tubes 20 change to a bending state illustrated in Fig. 8 from a straight state illustrated in Fig. 6. The glove-type power assist device of the present embodiment assists folding motion of fingers as illustrated in Figs. 4 and 5 taking advantage of this operating characteristics of the expanding and contracting tubes 20. Concrete configurations of the expanding and contracting tubes 20 is explained later.

### [Expanding and contracting tube]

Each expanding and contracting tube 20 of the glove-type power assist device of the present embodiment includes a tube main body 20a inflated by a pressure rise caused by a fluid provided to an inside thereof as illustrated in Fig. 6, a fluid injecting pipe 20b for injecting the fluid into the tube main body 20a. The tube main body 20a includes an inner tube 20a1, an outer tube 20a2,a3 for covering an outer side of the inner tube 20a1.

Among the inner tube 20a1 and the outer tube 20a2,a3, the inner tube 20a1 is made of an expandable and contractable material such as a rubber. The outer tube 20a2,a3 is configured by an expanding and contracting part 20a2 made of an expandable and contractable material such as a rubber, and an unexpanding and uncontracting part 20a3 made of an expandable and uncontractable material such as a cloth. The unexpanding and uncontracting part 20a3 is provided along a side of the tube main body 20a which is fixed to the finger inserting portion 11 of the glove 10. Therefore, a stretch of the expanding and contracting tube 20 along the longitudinal direction is prevented on a palm-side (the side of the expanding and contracting tube 20 which is fixed to the finger inserting portion 11) of the expanding and contracting tube 20.

The glove-type power assist device of the present embodiment is provided with the unexpanding and uncontracting part 20a3 to bend as follows. Namely, as illustrated in Fig. 6, in case a fluid is supplied to the expanding and contracting tubes 20 in a straight state (contracting state) and not in bending state, the expanding and contracting tubes 20 begin to bend to a palm-side (side on which the unexpanding and uncontracting portion 20a3 is provided) by expanding the back side (side on which the expanding and contracting portion 20a2 is provided) of the expanding and contracting tubes 20 more than the palm-side. This bending motions assist folding motions of fingers wearing the glove-type power assist device, as illustrated in Figs. 4 and 5.

By the way, in the glove-type power assist device of the present embodiment, as illustrated in Figs. 7 and 9, no gaps are provided between an inner periphery of the outer tube 20a2, 20a3 and an outer periphery of the inner tube 20a1 to have the inner periphery of the outer tube 20a2,20a3 and the outer periphery of the inner tube 20a1 always closely contacted. Therefore, the expanding and contracting tubes 20 begin to bend right after a provision of a fluid into the expanding and contracting tubes 20 to enhance a responsivity of the glove-type power assist device.

The expanding and contracting tubes 20 expand their diameter as they shift from a contracting state (unbending state) to an expanding state (bending state). Namely, a size of expanding and contracting tubes 20 is small in a state a fluid is not supplied to the expanding and contracting tubes 20. Therefore, the glove-type power assist device is downsized when storing or carrying it. In the glove-type power assist device of the present embodiment, diameter of the expanding and contracting tube 20 is approximately 5mm in contracting states (unbending state) and approximately 10mm in expanding states (bending state).

### [Fluid supplying member]

The fluid supplying member supplies a fluid to the expanding and contracting tubes 20. In the glove-type power assist device of the present embodiment, the fluid supplying member may be a member utilizing a compressor or a motor. However, in the glove-type power assist device of the present embodiment, the fluid supplying member includes a tank filled with a fluid to be supplied to expanding and contracting tubes 20, an on-off valve for opening and closing the tank, a valve controlling portion for controlling the opening or closing status of the on-off valve, and a portable battery (such as a battery or an electric cell) supplying a power to the on-off valve and the valve controlling portion. By adapting this, the fluid supplying member is downsized which is suitable for taking along, also vibrations and noises emitted from fluid supplying member are reduced, and also power consumption of the fluid supplying member is reduced.

Although a fluid filled into the tank of the fluid supplying member may be either gas or liquid, a liquefied carbon dioxide is preferably filled. By adapting this, a large amount of the liquefied carbon dioxide is provided to the expanding and contracting tubes 20 with the downsized tank. The liquefied carbon dioxide is easy to handle, safe and easy to obtain. Volume of the tank is 95ml in the glove-type power assist device of the present embodiment. The glove-type power assist device is able to perform grabbing motions approximately 200 times with this tank of said volume. This is sufficient volume considering that a person who often uses the glove-type power assist device conducts grabbing motions approximately 100 times a day.

### [Glove]

In the glove-type power assist device of the present embodiment, the finger inserting portions 11 of the glove 10 consist essentially of thumb inserting portion for inserting wearer's thumb, the forefinger inserting portion for inserting wearer's forefinger, the three fingers commonly inserting portion for inserting wearer's middle finger, ring finger and little finger, as illustrated in Fig. 1. Therefore, an operability of the glove-type power assist device is enhanced and the glove-type power assist device is easily worn. In addition, costs required for the glove-type power assist device are reduced because the configuration is simplified. For example, if the number of finger inserting portions is five, five expanding and contracting tubes 20 are needed. However, the number of expanding and contracting tube 20 become three at the minimum by adapting the three fingers commonly inserting portion. In the glove-type power assist device of the present embodiment, one expanding and contracting tube for assisting a thumb 21 is provided on the thumb inserting portion 11a, one expanding and contracting tube for assisting a forefinger 22 is provided on the forefinger inserting portion 11b, and two expanding and contracting tubes for assisting three fingers 23a,23b are provided on the three fingers commonly inserting portion 11c. Therefore, the number of the expanding and contracting tubes 20 adds up to four.

In the glove-type power assist device of the present embodiment, a cutline is provided on the glove to cover a portion from a base of a forefinger on a thumb side to an opening for wearing the glove, as illustrated in Fig. 2. A fastener 12 is provided for fastening the cutline. This makes it easy for those who have a handicap on fingers to wear the glove-type power assist device. Especially, a thumb is easily inserted into the thumb inserting portion 11a. Inserting the thumb into the thumb inserting portion 11a is most difficult. The glove-type power assist device of the present embodiment adapts a slide fastener as the fastener 12.

Substantially whole of the glove 10 is configured by an elastic base material 13 having elasticity as illustrated in Fig. 1. Portions made of the elastic base material 13 are cross hatched with smaller pitched hatching lines in Figs. 1 to 5. Elastic cloths used as sport inner wears are preferably used as the elastic base material 13. The glove-type power assist device is worn without giving uncomfortable feelings by adapting this. Non-slip portions covered with non-slip sheets 14 are provided on a palm side than the side lines L1 (Fig. 4) of the glove 10, as illustrated in Fig. 3. Rubber sheets are preferably used as the non-slip sheets 14. Portions provided with the non-slip sheets 14 are cross hatched with larger pitched hatching lines in Figs. 1 to 5.

In the glove-type power assist device of the present embodiment, only the unbending portions on a palm including the finger inserting portions not overlapping finger joints of a hand are covered with the non-slip sheets 14, as illustrated in Fig. 3. In other words, bending portions overlapping the finger joints of the finger inserting portions 11 and provided on the palm-side than the side lines L1 are configured as blank portions 16 not covered with the non-slip sheets 14. The elastic base material 13 is exposed from the blank portions 16. This facilitates the finger inserting portions 11 to easily bend and enhances an operability and a wear comfort of the glove-type power assist device.

In the glove-type power assist device of the present embodiment, unstretchable liner members 15 are fixed to portions, which are boundaries between the backside and the palm-side of the finger inserting portions 11 of the glove 10, along the side lines L1 of the finger inserting portions 11 as illustrated in Fig. 4. Therefore, the portions along the side lines L1 of the finger inserting portions 11 has a configuration in which a stretch in longitudinal direction is prevented. The unstretchable liner members 15 are illustrated with bold lines in Figs. 1 to 5. The unstretchable liner members 15 may be provided on one side of sides of either one of the finger inserting portions 11. In the glove-type power assist device of the present embodiment, as illustrated in Fig. 1, the unstretchable liner members 15 are fixed along both of the left and right sides of the forefinger inserting portion 11b and along both of the left and right sides of the three fingers insertion portion 11c.

The unstretchable liner members 15 are fixed in various manners. In the glove-type power assist device of the present embodiment, the unstretchable liner members 15 are fixed to the finger inserting portions 11 by sewing the stringlike unstretchable liner members 15 along the boundary between the elastic base material 13 and the non-slip sheets 14. Strings made of synthetic fibers are used as the unstretchable liner members 15. This makes bending centers of the finger inserting portions 11 and folding centers of wearer's finger joints more promptly and more securely correspond each other. Therefore, uncomfortable feelings given to a wearer is relieved.

### [Use]

Use of the glove-type power assist device of the present invention is not particularly limited. It is used for assisting a gripping power required for various motions in daily lives such as to hold a spoon, to hold a cup, to hold a pencil, also for rehabilitations for those who have handicaps on finger tips, trainings on how to use finger tips and stretches for preventing contracture. It is also used as a glove-type load applying device used for muscle trainings. Further, it is uses as a glove-type virtual experience device used for embodying virtual realities. Because the glove-type power assist device of the present invention smoothly assists finger motions of a hand of a wearer without giving a large strain on wearer's finger joints of the hand, and is easy to downsize and suitable for taking along, it is used for broad purposes as exemplified above.

A case where the glove-type power assist device assists folding motions of fingers of a hand is exemplified and explained so far. The glove-type power assist device is also used to assist stretching motions of fingers of a hand. Namely, if a fluid is discharged from the expanding and contracting tubes which are in bending state by being supplied with the fluid, the expanding and contracting tubes straighten from the bending state to assist stretching motions of fingers of a hand. However, in this case, large assisting power (operational ability) is not obtained in some cases. Therefore, it is preferable that two kinds of expanding and contracting tubes (first expanding and contracting tube and second expanding and contracting tube) are fixed along the back side of the common finger inserting portion, wherein the first expanding and contracting tube is configured so that a stretch in a longitudinal direction on a fixed side (palm-side) fixed with respect to the finger inserting portion is prevented (configured to have the same structure as the expanding and contracting tube 20 in Fig. 1), the second expanding and contracting tube is configured so that a stretch in the longitudinal direction on a side (back side) opposite to the fixed side fixed with respect to the finger inserting portion is prevented (configured to have the same structure as the expanding and contracting tube 20 in Fig. 1), an expanding and contracting tube to be supplied with the fluid from the tank (fluid supplying member) is interchanged between the first expanding and contracting tube and the second expanding and contracting tube according to a motion to be executed by the glove-type power assist device. Concretely, in case bending motions are executed, a fluid is supplied from the tank (fluid supplying member) to the first expanding and contracting tube while discharging a fluid from the second expanding and contracting tube. In case stretching motions are executed, a fluid is supplied from the tank (fluid supplying member) to the second expanding and contracting tube while discharging a fluid from the first expanding and contracting tube. By adapting this, a large operational ability is obtained for stretching motions of fingers of a hand is assisted as well as for folding motions of the fingers of the hand. This configuration is preferably applicable to all purposes explained above.

### Reference numerals

10 Glove
11 Finger inserting portion
11a Thumb inserting portion
11b Forefinger inserting portion
11c Three fingers commonly inserting portion
12 Fastener
13 Elastic base material
14 Non-slip sheet
15 Unstretchable liner member
16 Blank portion
20 Expanding and Contracting tube
20a Tube main body
20a1 Inner tube
20a2 Contracting part (outer tube)
20a3 Unexpanding and uncontracting part (outer tube)
20b Fluid injecting pipe
21 Expanding and contracting tube for assisting a thumb
22 Expanding and contracting tube for assisting a forefinger
23a Expanding and contracting tubes for assisting three fingers
23b Expanding and contracting tubes for assisting three fingers
L1 Side line

## Claims

1. A glove-type power assist device comprising:
(a) a glove (10) provided with finger inserting portions (11, 11a, 11b, 11c) for inserting fingers of a hand;
(b) expanding and contracting tubes (20, 21, 22, 23a, 23b) fixed to back side of the finger inserting portions (11, 11a, 11b, 11c) of the glove (10), a stretch along a longitudinal direction being restricted on a side fixed to the finger inserting portions (11, 11a, 11b, 11c), and
(c) a fluid supplying member supplying a fluid to the expanding and contracting tubes (20, 21, 22, 23a, 23b);
wherein the expanding and contracting tubes (20, 21, 22, 23a, 23b) are bent to a palm-side by stretching the back side of the expanding and contracting tubes (20, 21, 22, 23a, 23b) more than the palm-side to assist a folding motion of fingers inserted into the finger inserting portions (11, 11a, 11b, 11c),
wherein substantially whole of the glove (10) is configured by an elastic base material (13),
**characterized in that**
side lines (L1) are provided as boundaries between the back side and the palm-side of the finger inserting portions (11, 11a, 11b, 11c) in the position where the side lines (L1) overlap with lines connecting the folding points of wearer's finger joints when the finger inserting portions (11, 11a, 11b, 11c) are laterally seen,
the side lines (L1) have an unstretchable configuration in which a stretch to the longitudinal direction of the finger inserting portions (11, 11a, 11b, 11c) is prevented so that the bending centers of the expanding and contracting tubes more precisely correspond to the folding points of wearer's finger joints as centers.

2. The glove-type power assist device according to Claim 1, wherein the side lines (L1) are made unstretchable configuration by fixing unstretchable liner members (15) along the side lines (L1) of the finger inserting portions (11, 11a, 11b, 11c).

3. The glove-type power assist device according to any one of Claims 1 or 2, wherein unbending portions not overlapping the finger joints of the finger inserting portions (11, 11a, 11b, 11c) and provided on the palm-side are configured as non-slip portions covered with non-slip sheets (14), bending portions overlapping the finger joints of the finger inserting portions (11, 11a, 11b, 11c) and provided on the palm-side are configured as blank portions (16) not covered with the non-slip sheets (14) and exposing the elastic base material (13).

4. The glove-type power assist device according to any one Claims 1 to 3, wherein a cutline is provided on the glove (10) to cover a portion from a base of a forefinger on a thumb side to an opening for wearing the glove (10), a fastener (12) is provided for fastening the cutline.

5. The glove-type power assist device according to any one of Claims 1 to 4, wherein the finger inserting portions (11, 11a, 11b, 11c) consist essentially of a thumb inserting portion (11a) for inserting wearer's thumb, a forefinger inserting portion (11b) for inserting wearer's forefinger, a three fingers commonly inserting portion (11c) for inserting wearer's middle finger, ring finger and little finger.

6. The glove-type power assist device according to any one of Claims 1 to 5, wherein the fluid supplying member includes a tank whose capacity being equal to or below than 500ml and filled with a liquefied carbon dioxide, an on-off valve for opening and closing the tank, and a valve controlling portion for controlling the opening or closing status of the on-off valve.

## Patentansprüche

1. Bewegungshilfsvorrichtung in Handschuhform mit:
(a) einem Handschuh (10), versehen mit Finger-Einschub-Teilen (11, 11a, 11 b, 11 c) zum Einschieben von Fingern einer Hand;
(b) sich dehnenden und zusammenziehenden Schläuchen (20, 21, 22, 23a, 23b), befestigt an der Rückseite der Finger-Einschub-Teile (11, 11a, 11b, 11c) vom Handschuh (10), wobei die Streckung entlang der Längsrichtung an der Seite, an der die Finger-Einschub-Teile (11, 11a, 11b, 11c) befestigt sind, eingeschränkt ist, und
(c) einem Fluid-Zuführungs-Element zum Zuführen eines Fluids zu den sich dehnenden und zusammenziehenden Schläuchen (20, 21, 22, 23a, 23b);
wobei die sich dehnenden und zusammenziehenden Schläuche (20, 21, 22, 23a, 23b) zu der Handflächen-Seite durch Dehnen der Rückseite der sich dehnenden und zusammenziehenden Schläuche (20, 21, 22, 23a, 23b) mehr als die Handflächen-Seite gekrümmt werden, zum Unterstützen einer Biege-Bewegung der in die Finger-Einschub-Teile (11, 11a, 11b, 11c) eingeschobenen Finger,
wobei im Wesentlichen der gesamte Handschuh (10) durch ein elastisches Grundmaterial (13) ausgestaltet ist,
**dadurch gekennzeichnet, dass**
Seitenlinien (L1) als Abgrenzungen zwischen der Rückseite und der Handflächen-Seite der Finger-Einschub-Teile (11, 11a, 11b, 11c) in der Position bereitgestellt werden, in der die Seitenlinien (L1) mit Linien überlappen, die die Biege-Punkte der Finger-Gelenke des Trägers verbinden, wenn man die Finger-Einschub-Teile (11, 11a, 11b, 11c) seitlich beobachtet, die Seitenlinien (L1) eine nicht-dehnbare Ausgestaltung aufweisen, wobei eine Streckung zu der Längsrichtung der Finger-Einschub-Teile (11, 11 a, 11 b, 11 c) verhindert wird, sodass die Biege-Zentren der sich dehnenden und zusammenziehenden Schläuche präziser den Biege-Punkten der Finger-Gelenke des Trägers als Zentren entsprechen.

2. Bewegungshilfsvorrichtung in Handschuhform nach Anspruch 1, wobei die Seitenlinien (L1) in einer nicht-dehnbaren Ausgestaltung durch Befestigen nicht-dehnbarer Auskleidungs-Elemente (15) entlang den Seitenlinien (L1) der Finger-Einschub-Teile (11, 11a, 11b, 11c) hergestellt worden sind.

3. Bewegungshilfsvorrichtung in Handschuhform nach einem von Ansprüchen 1 oder 2, wobei sich nicht krümmende Teile, die die Finger-Gelenke der Finger-Einschub-Teile (11, 11a, 11b, 11c) nicht überlappen und an der Handflächen-Seite vorgesehen sind, als nicht rutschende Teile, bedeckt mit nicht rutschenden Folien (14), ausgelegt sind, sich krümmende, die Finger-Gelenke der Finger-Einschub-Teile (11, 11a, 11b, 11c) überlappende, und auf der Handflächen-Seite vorgesehene Teile als freie Bereiche (16) ausgelegt sind, die nicht mit den nicht rutschenden Folien (14) bedeckt sind und das elastische Grundmaterial (13) freiliegen lassen.

4. Bewegungshilfsvorrichtung in Handschuhform nach einem von Ansprüchen 1 bis 3, wobei ein Einschnitt an dem Handschuh (10) vorgesehen ist, zum Bedecken eines Teils von dem Unterteil des Zeigefingers an der Daumen-Seite für eine Öffnung zum Tragen des Handschuhs (10), ein Verschluss (12) zum Verschließen des Einschnitts vorgesehen ist.

5. Bewegungshilfsvorrichtung in Handschuhform nach einem von Ansprüchen 1 bis 4, wobei die Finger-Einschub-Teile (11, 11a, 11b, 11c) im Wesentlichen aus einem Daumen-Einschub-Teil (11a) zum Einschieben des Daumens des Trägers, einem Zeigefinger-Einschub-Teil (11 b) zum Einschieben des Zeigefingers des Trägers, einem für drei Finger gemeinsamen Einschub-Teil (11c) zum Einschieben von Mittel-Finger, Ring-Finger und kleinem Finger des Trägers bestehen.

6. Bewegungshilfsvorrichtung in Handschuhform nach einem von Ansprüchen 1 bis 5, wobei das Fluid-Zuführungs-Element einen Behälter enthält, dessen Kapazität gleich 500 ml oder weniger ist und der mit verflüssigtem Kohlenstoffdioxid gefüllt ist, einem Ein-Aus-Ventil zum Öffnen und Schließen des Behälters und einem Ventil-Steuer-Teil zum Steuern des Öffnungs- und Schließ-Status des Ein-Aus-Ventils.

## Revendications

1. Dispositif assisté de type gant comprenant :
(a) un gant (10) pourvu de portions d'insertion de doigt (11, 11a, 11b, 11c) pour insérer les doigts d'une main ;
(b) des tubes de dilatation et de contraction (20, 21, 22, 23a, 23b) fixés au côté arrière des portions d'insertion de doigt (11, 11a, 11b, 11c) du gant (10), une étendue le long d'une direction longitudinale étant limitée sur un côté fixé aux portions d'insertion de doigt (11, 11a, 11b, 11c), et
(c) un élément de fourniture de fluide fournissant un fluide aux tubes de dilatation et de contraction (20, 21, 22, 23a, 23b) ;
dans lequel les tubes de dilatation et de contraction (20, 21, 22, 23a, 23b) sont courbés vers un côté paume en étirant le côté arrière des tubes de dilatation et de contraction (20, 21, 22, 23a, 23b) davantage que le côté paume pour aider un mouvement de pliage de doigts insérés dans les portions d'insertion de doigt (11, 11a, 11b, 11c),
dans lequel essentiellement l'ensemble du gant (10) est configuré par un matériau de base élastique (13),
**caractérisé en ce que**
des lignes latérales (L1) sont prévues comme limites entre le côté arrière et le côté paume des portions d'insertion de doigt (11, 11a, 11b, 11c) dans la position où les lignes latérales (L1) chevauchent des lignes connectant les points de pliage des articulations de doigt du porteur lorsque les portions d'insertion de doigt (11, 11a, 11b, 11c) sont vues latéralement, les lignes latérales (L1) ont une configuration non étirable dans laquelle un étirement vers la direction longitudinale des portions d'insertion de doigt (11, 11a, 11b, 11c) est empêché de sorte que les centres de courbure des tubes de dilatation et de contraction correspondent plus précisément aux points de pliage des articulations de doigt du porteur comme centres.

2. Dispositif assisté de type gant selon la revendication 1, dans lequel les lignes latérales (L1) sont réalisées configuration non étirable en fixant des éléments de doublure non étirables (15) le long des lignes latérales (L1) des portions d'insertion de doigt (11, 11a, 11b, 11c).

3. Dispositif assisté de type gant selon l'une quelconque des revendications 1 ou 2, dans lequel des portions de redressement ne chevauchant pas les articulations de doigt des portions d'insertion de doigt (11, 11a, 11b, 11c) et prévues sur le côté paume sont configurées comme portions antidérapantes recouvertes de feuilles antidérapantes (14), des portions de courbure chevauchant les articulations de doigt des portions d'insertion de doigt (11, 11a, 11b, 11c) et prévues sur le côté paume sont configurées comme portions vierges (16) non recouvertes des feuilles antidérapantes (14) et exposant le matériau de base élastique (13).

4. Dispositif assisté de type gant selon l'une quelconque des revendications 1 à 3, dans lequel une ligne de découpe est prévue sur le gant (10) pour recouvrir une portion d'une base d'un index sur un côté pouce à une ouverture pour porter le gant (10), un dispositif de fixation (12) est prévu pour fixer la ligne de découpe.

5. Dispositif assisté de type gant selon l'une quelconque des revendications 1 à 4, dans lequel les portions d'insertion de doigt (11, 11a, 11b, 11c) sont essentiellement constituées d'une portion d'insertion de pouce (11a) pour insérer le pouce du porteur, d'une portion d'insertion d'index (11b) pour insérer l'index du porteur, d'une portion d'insertion commune de trois doigts (11c) pour insérer le majeur, l'annulaire et l'auriculaire du porteur.

6. Dispositif assisté de type gant selon l'une quelconque des revendications 1 à 5, dans lequel l'élément de fourniture de fluide inclut un réservoir dont la capacité est inférieure ou égale à 500 ml et rempli d'un dioxyde de carbone liquéfié, une soupape marche-arrêt pour ouvrir et fermer le réservoir, et une portion de commande de soupape pour commander le statut d'ouverture ou de fermeture de la soupape marche-arrêt.
